# EUROPÄISCHE PATENTSCHRIFT

(11) **EP 1 893 146 B1**
(45) Veröffentlichungstag und Bekanntmachung des Hinweises auf die Patenterteilung: **08.03.2017**
(21) Anmeldenummer: 06753832.2
(22) Anmeldetag: 24.05.2006
(51) Int. Cl.: A61F 13/00, A61F 13/02

(54) **VORRICHTUNG MIT WUNDDOKUMENTATIONSHILFE**
DEVICE COMPRISING A WOUND DOCUMENTATION AID
DISPOSITIF DOTE D'UN AUXILIAIRE DE DOCUMENTATION DE BLESSURE

(30) Priorität: 15.06.2005 DE 102005027656
(43) Veröffentlichungstag der Anmeldung: 05.03.2008
(73) Patentinhaber: Paul Hartmann AG, 89522 Heidenheim (DE)
(72) Erfinder: ZWIBEL, Frank, 89359 Kötz (DE); GARRIGOS SISTARE, Gema, E-08034 Barcelona (ES); EFFING, Jochem, 65779 Kelkheim-Fischbach (DE); ECKSTEIN, Axel, 89522 Heidenheim (DE)
(86) Internationale Anmeldenummer: PCT/EP2006/004933
(87) Internationale Veröffentlichungsnummer: WO 2006/133794

(56) Entgegenhaltungen:
- EP-A1- 0 424 165
- EP-A1- 0 630 629
- EP-A1- 0 640 332
- EP-A2- 0 341 045
- EP-A2- 0 730 845
- WO-A2-90/03155
- US-A- 5 265 605

## Beschreibung

Die Erfindung betrifft eine Vorrichtung, die eine Wundauflage und eine Wunddokumentationshilfe zur Dokumentation von charakteristischen Daten einer Wunde umfasst.

Hilfsmittel zur Wunddokumentation von Wunden sind in vielfältiger Weise bekannt. Die meisten auch durch die Patentliteratur bekannten Wunddokumentationshilfen sind zusätzliche Hilfsmittel, die keine direkte therapeutische Maßnahme ermöglichen. So beschreibt z.B. die EP 730 845 A2 eine Vorrichtung, die aus zwei voneinander trennbaren, übereinander liegenden, transparenten Blättern bestehen. Auf einem der Blätter ist ein Gitter zur Ermittlung und Erfassung der Größe einer Wunde aufgebracht. Bei der Verwendung dieser Vorrichtung werden die beiden Blätter über einer Wunde positioniert und die Erfassung und Markierung der Wundgröße auf dem mit dem Gitter versehenden Blatt vorgenommen. Nach der Aufzeichnung wird das die Markierungen tragende Blatt von dem während der Aufzeichnung auf der Wunde liegendem Blatt entfernt. Die Aufzeichnungen können kontaminationsfrei in einer Patientenmappe aufbewahrt werden.

Weiterhin ist mit der EP 640 332 B1 eine sterile Verpackung für Wundauflagen bekannt, die zur Wunddokumentation transparente Bereiche aufweist und auf der sterilen Innenseite beschreibbar ist. In der Verpackung ist eine Wundauflage lose eingebracht, die während der Aufzeichnung von Daten, wie z.B. Wundgröße in einem sterilen Bereich außerhalb der Verpackung aufbewahrt werden muss.

Mit der deutschen Anmeldung DE 100 08 105 A1 wird ebenfalls eine Vorrichtung zur Größenbestimmung von Wundflächen beschrieben, die eine transparente Folie mit darauf befindlichen Hilfslinien aufweist. In dem mit den Hilfslinien versehenen Bereich der Folie weist die Vorrichtung eine nicht klebende oder nicht haftende Oberfläche auf. Vorzugsweise kann die Vorrichtung zwei Folien umfassen, wobei die zweite Folie bei Anwendung und nach Aufzeichnung der Dokumentation auf der ersten Folie von der ersten Folie ablösbar ist und lediglich die erste Folie aufbewahrt wird.

Darüber hinaus ist mit der europäischen Patentschrift EP 341 045 B1 ein klebender, transparenter oder transluzenter Folienverband beschrieben, der eine transparente Folie umfasst, die ihrerseits Bezugszeichen umfasst. Diese Bezugszeichen können zum Beobachten der Wunde verwendet werden.

Des Weiteren wird mit der EP 630 629 B1 eine mehrschichtige, transparente Hydrogel-Wundauflage beschrieben, die eine Wundkontaktschicht aus einem Polyurethan-Hydrogel, eine poröse Trägerschicht und einen dünnen Film als Abdeckschicht umfasst. Zwischen dem Film und dem Hydrogel kann vorzugsweise eine weitere Schicht eingebracht werden, die zur Wundbeobachtung ein Gitteraufdruck trägt, der die Wundbeobachtung und das Vermessen der Wunde erleichtern soll, ohne die applizierte Wundauflage von der Wunde zu entfernen. Diese Wunddokumentationshilfe kann jedoch nicht von der Wundauflage entfernt werden, ohne die Wundauflage selbst zu entfernen.

Daher ist es eine Aufgabe der vorliegenden Erfindung eine Vorrichtung bereitzustellen, die sowohl eine therapeutische Maßnahme als auch die Dokumentation des Wundzustandes oder die Dokumentation der therapeutischen Maßnahme selbst im Routinebetrieb ermöglicht und sicherstellt. Die Vorrichtung sollte sicher in der Anwendung sein und eine zuverlässige Dokumentation der therapeutischen Maßnahme ermöglichen. Weiterhin sollte mit dieser Vorrichtung ein Mittel zur Verfügung gestellt werden, das die Dokumentation eines Wundheilungsprozesses ohne Reizung der Wunde gewährleistet. Darüber hinaus ist es Aufgabe der vorliegenden Erfindung ein Verfahren zur Herstellung einer solchen Vorrichtung und eine Methode zur Wunddokumentation bereit zustellen.

Gelöst werden diese Aufgaben durch eine Vorrichtung gemäss Anspruch 1.

Ein Vorteil dieser Vorrichtung besteht in ihrer Anwendung. Mit dieser Vorrichtung wird dem Anwender in sehr einfacher Weise ein Mittel an die Hand gegeben, das die Durchführung einer therapeutischen Maßnahme ermöglicht und gleichzeitig ein Hilfsmittel zur Dokumentation des Ist-Zustandes der zu therapierenden Wunde ermöglicht. Da die Wunddokumentationshilfe mit der Wundauflage lösbar verbunden ist, kann mit dieser Vorrichtung die Möglichkeit einer umfassenden Datensammlung zu der therapeutischen Maßnahme mit einem standardisierten Verfahren zur Dokumentation der zu therapierenden Wunde bereitgestellt werden. Hierbei kann die Wunddokumentationshilfe selbst archiviert werden. Durch die Beabstandung der Dokumentationshilfe von der Wunde mittels der Wundkontaktschicht ist darüber hinaus bei der Anwendung eine kontaminationsfreie Aufzeichnung und Dokumentation der Wundparameter möglich, die zudem ohne eine Reizung der Wunde selbst erfolgen kann.

Im Kontext dieser Anmeldung soll hierbei und im Folgenden unter Dokumentationshilfe jedwedes Mittel verstanden sein, das die Wahrnehmung und Dokumentation mindestens einer Kenngröße einer Wunde erlaubt. Hierzu zählen insbesondere die Parameter der Wunde, die den Zustand der Wunde beschreiben, wie zum Beispiel Farbe und Aussehen, Größe, Alter oder Wundheilungsstadium. Unter Dokumentation ist hierbei das zeitweise sammeln und festhalten von Daten zu verstehen. Des Weiteren soll unter einer die Wundheilung fördernde Wundkontaktschicht eine Schicht verstanden sein, die über ein bloßes Festhalten der Trägerschicht und damit ein zeitweises Abdecken der Wunde mittels der Trägerschicht einen zusätzlichen Beitrag zur Wundheilung liefert. Dies können unterschiedliche Eigenschaften sein wie zum Beispiel das Aufsaugen von Wundsekret, die Bereitstellung oder Abgabe von Wundheilungsfördernden oder -unterstützenden Substanzen oder das Bereitstellen von Wundheilungsfördernden oder -unterstützenden Medien.

In einer zweiten bevorzugten Ausführungsform weist die Vorrichtung eine Dokumentationshilfe auf, die mindestens eine Folie umfasst. Hierbei ist es vorteilhaft, wenn die im anwendungsgerechten Zustand wundabgewandte Seite der Folie mit einem üblichen Schreibgerät wie zum Beispiel einem Permanent-Marker beschreibbar ist. So kann zum Beispiel eine Kennzeichnung der durch visuelle Beobachtung ermittelten Wundflächen oder anderer charakteristischen Daten der Wunde erfolgen.

In einer besonders bevorzugten Ausführungsform umfasst die Wunddokumentationshilfe mindestens zwei Wunddokumentationsfelder zur Speicherung von unterschiedlichen Informationen. Somit kann mit dieser Vorrichtung eine Wundauflage bereitgestellt werden, die neben der therapeutischen Maßnahme die Möglichkeit einer umfassenden Datensammlung zu der therapeutischen Maßnahme und dem Zustand der Wunde erlaubt. Zum Beispiel kann in ein Dokumentationsfeld z.B. die Speicherung der Daten zu der Art, Datum bzw. Zeitraum oder Mittel der Behandlung erfolgen, wogegen in dem zweiten Dokumentationsfeld z.B. die Speicherung der Daten über Zustand der Wunde zum Zeitpunkt des Behandlungsbeginns erfolgen kann. Diese Aufteilung ermöglicht somit ein standardisiertes Verfahren zur Dokumentation der zu therapierenden Wunde und der Therapie selbst.

Des Weiteren ist vorgesehen, dass die Dokumentationshilfe einer erfindungsgemäßen Vorrichtung unterschiedliche Hilfslinien in Form von geometrischen Figuren aufweist. Insbesondere sind die Hilfslinien auf die von der Dokumentationshilfe umfassten beschreibbaren Folie aufzubringen. Beispielsweise können Rechteckgitter oder auch konzentrische Kreise oder Ellipsen auf der Folie oder der Dokumentationshilfe angebracht sein. Mit Hilfe dieser Hilfslinien kann die Wundgröße, die unterschiedlichen Flächenanteile an unterschiedlichen Geweben innerhalb der Wunde oder Heilungszuständen von Wundbereichen innerhalb der Wunde leichter ermittelt werden. Hierbei kann es vorteilhaft sein, die unterschiedlichen Hilfslinien durch verschiedene Farbgestaltung oder Liniengestaltung, beispielsweise gepunktet, gestrichelt oder durchgehend, voneinander unterscheidbar zu machen.

Um die Ermittlung des zu einer bestimmten geometrischen Figur gehörenden Flächeninhaltes zu erleichtern, kann weiterhin vorgesehen sein, den Hilfslinien oder zumindest einigen dieser Linien Zahlenangaben oder Maßangaben zuzuordnen, aus denen der jeweilige Flächeninhalt hervorgeht. Zum Beispiel können solche Angaben zusammen mit den Hilfslinien aufgebracht werden. Diese Angaben können Zahlen- oder Buchstabencodes sein, vorzugsweise farblich auf die dazugehörigen Linien abgestimmt, welche es ermöglichen, die Flächenangaben aus dazugehörigen Tabellen zu entnehmen.

Als Folien sind besonders transparente oder transluzente Folienmaterialien vorgesehen. Insbesondere kommen hierbei transparente Folien zur Verwendung, die aus Polyester, Co-Polyester, Polyethylen, Polypropylen, Polyvinylchlorid, Polystyrol, Polyamid, Polykarbonat, Celluloseester, Ethylenvinylacetat, Polyvinylacetat, Polyvinylalkohol und/oder Mischungen hiervon gefertigt werden. Besonders bevorzugt sind Folien aus transparentem Polyester oder Polyethylen oder Polypropylen oder Ethylenvinylacetat. Die Folien dürfen eine bestimmte Dicke nicht überschreiten, da ansonsten bei der Archivierung ein zu großer Platzbedarf besteht. Es hat sich als besonders vorteilhaft erwiesen, wenn die Folien eine Dicke von 10 bis 80 µm, insbesondere von 20 bis 60 µm und ganz bevorzugt von 20 bis 40 µm aufweisen.

Es kann weiterhin vorgesehen sein, als Dokumentationshilfe eine mehrlagige Folie oder einen Folienverbund zu verwenden. Diese mehrlagigen Folien besitzen den Vorteil, dass ein eventueller Aufdruck auf einer ersten Lage der Folie durch eine zweite Lage Folie abgedeckt werden kann. Somit kann eine Markierung mittels eines Permanent-Markers auf einer äußeren Oberfläche der Folie erfolgen, die eine gleichmäßige und einheitliche Form aufweist. Es kann weiterhin vorgesehen sein, dass die Folie als ganzes eingefärbt ist, derart, dass sie auf jeden Fall transparent bzw. transluzent ist.

Die Vorrichtung weist eine Dokumentationshilfe in Form einer Folie auf, die ihrerseits mindestens einen klebend ausgerüsteten Bereich beispielsweise in Streifenform oder als Klebepunkte aufweist. Mit Hilfe dieses klebenden Bereiches kann die Dokumentationshilfe zum Beispiel in einer Patientenakte fixiert werden und somit eine dauerhafte Archivierung der aufgezeichneten Maßnahmen und Daten erfolgen. Es kann auch vorgesehen sein, dass die Dokumentationshilfe mindestens einen klebenden Bereich aufweist, der mittels eines Abdeckstreifens abgedeckt ist. Da die Wunddokumentationshilfe als ganzes von der Wundauflage lösbar und auch archivierbar ist, kann somit zum Beispiel eine vergleichende Messung der Wundgröße zu unterschiedlichen Zeitpunkten bzw. bei jedem Verbandwechsel erfolgen.

Weiterhin kann eine erfindungsgemäße Vorrichtung eine Wunddokumentationshilfe aufweisen, die mindestens eine Griffleiste aufweist. Diese Griffleiste erleichtert dem Anwender das Ergreifen und Handhaben der Dokumentationshilfe bei der Anwendung der Vorrichtung und der Dokumentation der Der mittels eines Abdeckstreifens abgedeckte, klebend ausgerüstete Bereich der Dokumentationshilfe ist als Griffleiste zu verwenden Hierbei ist insbesondere vorgesehen, dass der klebende Bereich entlang eines Randes der Dokumentationshilfe angebracht ist.

Als Trägerschicht der Wundauflage einer erfindungsgemäßen Vorrichtung können verschiedene Materialien eingesetzt werden. Üblicherweise werden in Wundauflagen textile Trägermaterialien, Nonwoven, Polymerfilme oder Polymerschäume eingesetzt. Als Trägerschicht der Wundauflage einer erfindungsgemäßen Vorrichtung können insbesondere Polymerfilme eingesetzt werden. Ganz besonders bevorzugt sind Polymerfilme, die eine hohe Wasserdampfdurchlässigkeit aufweisen. Hierzu sind besonders Filme geeignet, die aus Polyurethan, Polyetherurethan, Polyesterurethan, Polyether-Polyamid-Copolymeren, Polyacrylat oder Polymethacrylat gefertigt werden. Insbesondere ist als Polymerfilm ein Polyurethanfilm, Polyesterurethanfilm oder Polyetherurethanfilm bevorzugt. Ganz besonders sind aber auch solche Polymerfilme bevorzugt, die eine Dicke von 15 bis 50 µm, insbesondere 20 bis 40 µm und ganz besonders bevorzugt von 25 bis 30 µm aufweisen. Die Wasserdampfdurchlässigkeit des Polymerfilms der Wundauflage weist vorzugsweise mindestens 750 g/ m²/ 24 Std., insbesondere mindestens 1000 g/ m²/ 24 Std. und ganz besonders bevorzugt mindestens 2000 g/ m²/ 24 Std. auf (gemessen nach DIN 13726).

Um eine anwendungssichere Vorrichtung bereit zu stellen, muss eine genaue Abstimmung der verwendeten Materialien erfolgen. Insbesondere müssen im Fall einer Folie als Dokumentationshilfe die verwendeten Materialien der Trägerschicht und der Folie hinsichtlich ihrer Release-Eigenschaften abgestimmt werden. Diese durch zusätzliche Mittel einstellbaren Release-Eigenschaften beruhen auf den Kräften, die zwischen zwei verwendeten Materialien herrschen. So kann zum Beispiel durch eine gezielte Oberflächenbehandlung eines Materials eine anziehende oder eine abstoßende Wirkung auf ein zweites mit diesem ersten Material zusammen zu bringendes Material eingestellt werden. Eine Oberflächenbehandlung, die eine anziehende Wirkung zwischen zwei Materialien hervorruft, kann zum Beispiel durch einen zusätzlichen adhäsiven Auftrag, eine statische Aufladung oder durch ein Verschmelzen der beiden zusammenzubringenden Materialien erfolgen. Eine abstoßende Wirkung kann zum Beispiel durch eine zusätzliche Schicht auf einem Material aus Silikon- oder Fluorverbindungen hervorgerufen werden. Als Release-Kraft (Abziehkraft) wird dabei eine solche Kraft verstanden, die notwendig ist zwei Materialien voneinander zu trennen (gemessen nach DIN 53530).

In einer weiteren Ausführungsform des erfindungsgemäßen Folienverbandes sind diese Release-Eigenschaften so eingestellt, dass ein Ablösen der Dokumentationshilfe in Form einer Folie von der Trägerschicht ohne großen Kraftaufwand mit der Hand erfolgen kann. Insbesondere ist die Release-Kraft so eingestellt, dass im applizierten Zustand der Vorrichtung ein Ablösen der Folie von der Trägerschicht erfolgen kann, ohne dass ein Verrutschen oder Ablösen der Wundauflage von der applizierten Stelle erfolgt. Die Haftung der Folie auf der Trägerschicht beträgt dabei vorzugsweise 0,01 bis 0,5 N/ 25 mm, ganz besonders bevorzugt 0,01 bis 0,1 N/ 25 mm, gemessen nach DIN 53530. Hierzu wird das Polymermaterial, aus dem die Dokumentationsfolie vorzugsweise besteht, direkt bei der Herstellung der Trägerschicht aufgebracht, beziehungsweise die Trägerschicht direkt auf der Dokumentationsfolie erzeugt. Hierbei können alle üblichen Verfahren zur Filmherstellung angewendet werden zum Beispiel durch Giessen, Rakeln, Extrudieren oder andere bekannte Methoden der Film- oder Folienherstellung. Falls notwendig, kann die Dokumentationsfolie auf der Beschichtungsseite aufgeraut oder einer anderen haftungsfördernden Behandlung unterworfen werden. Auch eine adhäsionsfördemde Beschichtung kann vorteilhaft sein.

In einer weiter bevorzugten Ausführung weist die Vorrichtung eine Dokumentationshilfe in Form einer Folie auf, die mittels eines Haftklebemittels mit der Trägerschicht verbunden ist. Dabei kann vorgesehen sein, dass die Folie mindestens einen Randbereich aufweist, der mit einem Ablösestreifen ausgestattet ist, so dass dieser Randbereich gleichzeitig als Griffleiste beim Entfernen der Folie von der Wundauflage fungieren kann. Hierbei ist darauf zu achten, dass der Haftkleber bei der Entfernung der Dokumentationshilfe nicht auf der Trägerschicht verbleibt. Der Haftkleber muss vollständig mit der Dokumentationshilfe entfernt werden.

In einer alternativen Ausgestaltung der Erfindung weist die Vorrichtung eine Dokumentationshilfe auf, die die gleiche Größe wie die Trägerschicht besitzt, das heißt, dass die Dokumentationshilfe und die Wundauflage dieselbe Umfangsbegrenzung aufweisen.

Darüber hinaus kann vorgesehen sein, dass eine erfindungsgemäße Wundauflage als so genanntes Inseldressing vorliegt. Hierbei weist die Wundkontaktschicht eine kleinere Auflagefläche auf als die Trägerschicht. Auf der der Dokumentationshilfe gegenüberliegenden zweiten Seite der Trägerschicht kann in dieser Form ein Haftklebemittel aufgebracht sein. Dieser Auftrag kann sowohl vollflächig als auch diskontinuierlich oder nur in bestimmten Bereichen erfolgen. Bei dem verwendeten Klebemittel kann es sich um einen üblichen Haftkleber, insbesondere um einen Acrylathaftkleber oder einen druckempfindlichen Haftkleber auf Basis von Polyurethanen handeln. Bevorzugt handelt es sich um Gelhaftkleber, insbesondere auf Basis von Polyurethanen, insbesondere wässrigen Polyurethanen. Ganz besonders bevorzugt handelt es sich um Hydrogelhaftkleber, insbesondere auf Basis von wässrigen Acrylaten.

In einer weiteren alternativen Ausgestaltung der Erfindung weist die Vorrichtung eine Dokumentationshilfe auf, die nicht transparent, das heißt, die opak ist. Eine solche Dokumentationshilfe kann zum Beispiel ein einfaches Etikett sein. Mit diesem Etikett kann alleine eine Dokumentation des angewendeten Produktes zu einem gegebenen Zeitpunkt bei einem bestimmten Patienten erfolgen, indem das Etikett direkt bei der Anwendung der erfindungsgemäßen Vorrichtung von der Vorrichtung gelöst wird und in eine Patientenmappe eingebracht wird. Diese Dokumentation kann unter gegebenenfalls gleichzeitiger Vermerkung des Ist-Zustandes der Wunde bei der Anwendung der Vorrichtung beispielsweise unter Kennzeichnung wie Ankreuzen von Wahlfeldem erfolgen. Vorzugsweise weist dieses Etikett einen sandwichartigen Aufbau auf, das heißt, dass das Etikett selbst einen vielschichtigen Aufbau aufweist. So kann zum Beispiel ein Papier-Etikett vorgesehen sein, das auf einer ersten Seite einen Aufdruck mit Informationen zu dem angewendeten Produkt und gegebenenfalls Wahlfeldern aufweist. Auf der zweiten Seite dieses Etikettes ist ein für Etiketten übliches Haftklebemittel aufgebracht. Dieses Etikett ist mit seiner klebend ausgerüsteten Seite auf ein zum Beispiel silikonisiertes Releasepapier aufgebracht, das seinerseits auf der dem Etikett gegenüberliegenden Seite selbstklebend ausgerüstet ist. Ein solches Etikett kann in einer ganz besonders bevorzugten Ausführung auf einer weiteren Folie aufgebracht sein, die ihrerseits lösbar mit einer Wundauflage und insbesondere lösbar mit einer Trägerschicht einer Wundauflage verbunden ist. Dem gemäß weist eine erfindungsgemäße Vorrichtung eine Dokumentationshilfe umfassend eine Folie auf, die ihrerseits ein ablösbares Etikett umfasst. Es kann jedoch auch vorgesehen sein, dass das Etikett mit einer Releaseschicht mittels der Releaseschicht direkt mit der Wundauflage lösbar verbunden ist.

Gemäß einer Weiterführung der Erfindung kann die Vorrichtung eine Trägerschicht aufweisen, die vollflächig mit einem Haftklebemittel beschichtet ist. Die Wasserdampfdurchlässigkeit der mit dem Haftklebemittel versehenen Trägerschicht beträgt dabei vorzugsweise mindestens 1000 g/ m²/24 Std., besonders bevorzugt mindestens 1200 g/m²/24 Std. und ganz besonders bevorzugt mindestens 2000 g/ m²/ 24 Std. (gemessen nach DIN EN 13726).

Eine erfindungsgemäße Vorrichtung kann in jeder geometrischen Form beispielsweise in dreieckiger, runder, ovaler oder quadratischer, rechteckiger oder jeder symmetrischen oder unsymmetrischen Form vorliegen. Hierbei ist es besonders vorteilhaft, wenn die Wundauflage und die Dokumentationshilfe dieselben Umfangsbegrenzungen aufweisen, das heißt, dass die Wundauflage und die Dokumentationshilfe gleich groß sind.

Als Wundkontaktschicht kann jede heute in der modernen Wundbehandlung übliche Wundkontaktschicht Verwendung finden. Hierbei sind insbesondere solche Wundkontaktschichten zu nennen, die in der feuchten Wundtherapie angewendet werden.

Damit kann eine erfindungsgemäße Vorrichtung insbesondere zur Herstellung eines Mittels zur Behandlung von Wunden, chronischen Wunden oder Hautschädigungen verwendet werden. Ganz besonders sind solche Wundkontaktschichten bevorzugt, die sowohl Wundsekret aufnehmen und somit absorbierend wirken als auch Feuchtigkeit an die Wunde abgeben. Verwendet werden erfindungsgemäss solche Wundkontaktschichten, die transparent oder transluzent sind. Gemäß einer besonderen Ausgestaltung der vorliegenden Erfindung umfasst die Vorrichtung eine Wundkontaktschicht, die mindestens ein Hydrogel, ein Hydrocolloid und/ oder einen Schaum insbesondere einen Polyurethanschaum umfasst.

Gemäß der vorliegenden Erfindung soll unter einem Hydrogel eine Zusammensetzung verstanden sein, die sich durch einen hohen Wassergehalt auszeichnet. Diese Hydrogele sind besonders als Wundkontaktschicht geeignet. Bezogen auf das Gesamtgewicht der Hydrogele weisen besonders bevorzugte Wundkontaktschichten einen Wassergehalt von 50 bis 95 Gew.-% auf. Dadurch sind diese Wundkontaktschichten besonders in der Lage Feuchtigkeit an eine Wunde abzugeben und Schorf, Nekrosen und andere Beläge in einer Wunde aufzuweichen und abzulösen. Diese Hydrogel umfassenden Wundkontaktschichten unterstützen damit das autolytische Debridment und sind zum Feuchthalten schwächer sezemierender Wunden geeignet. Damit sind erfindungsgemäße Vorrichtungen besonders zur Behandlung oberflächiger Wunden, wie Schürfwunden oder Wunden durch Spalthautentnahmen als auch bei chronischen Wunden wie Dekubitalgeschwüren oder Ulcera cures geeignet.

Ein bevorzugtes Hydrogel weist vorzugsweise eine wässerige, gelartige jedoch in sich zusammenhaltende Phase auf. Die gelartige Konsistenz des Hydrogels erzeugt eine Bindung zwischen der Wundauflage und der Wunde beziehungsweise die der Wunde umgebenden Haut, ohne dass es zu einer tatsächlichen Anhaftung der Wundauflage mit der Wunde kommt, die ein empfindliches Zellgewebe bei der Entfernung der Wundauflage beschädigen würde. Ein Vorteil des gelartigen Hydrogels ist, dass sich seine Qualität und der Zusammenhalt bei Absorption der Wundflüssigkeiten im Laufe der Behandlung kaum ändert. Darüber hinaus erlaubt es eine saubere und vollständige Entfernung der Wundauflage, wenn die Wunde heilt oder der Verband gewechselt wird. Ein solches Hydrogel wird zum Beispiel mit der EP 426 422 B1 beschrieben.

Ein weiterhin bevorzugtes Hydrogel ist ein Polyurethan-Hydrogel. Dieses Polyurethan-Hydrogel umfasst eine wässrigen Mischung, die 0 bis 90 Gew.-% mehrwertigen Alkohol, 6 bis 60 Gew.-% Präpolymer mit aliphatischen Diisocyanat-Gruppen, 4 bis 40 Gew.-% Polyamin auf Polyethylenoxidbasis, bis zu 2 Gew.-% Natriumchlorid und 50 bis 95 Gew.-% Wasser umfasst. Eine bevorzugte Hydrogelzusammensetzung zur Bildung der Hydrogelwundkontaktschicht umfasst 15 bis 30 Gew.-% mehrwertigen Alkohol ausgewählt aus einer Gruppe bestehend aus Polypropylenglycol, Polyethylenglycol und/ oder Glycerin, 5 bis 25 Gew.-% Präpolymer mit Isophorondüsocyanat-Enden, etwa 5 bis 20 Gew.-% Diamin auf Polyethylenoxidbasis, bis zu 2 Gew.-% eines Salzes und 50 bis 90 Gew.-% Wasser. Am bevorzugtesten umfasst das Hydrogel 15 bis 20 Gew.-% Polypropylenglycol und/ oder Glycerin, 5 bis 20 Gew.-% Präpolymer mit Isophorondiisocyanat-Enden, 5 bis 15 Gew.-% Diamin auf Polyethylenoxidbasis, bis zu 1 Gew.-% eines Salzes und 50 bis 80 Gew.-% Wasser. Das Hydrogelmaterial stellt ein biokompatibles, nicht reizendes, flüssigkeitsabsorbierendes, vor Bakterien schützendes, polsterndes, hautartiges Medium bereit, das insbesondere als Wundkontaktschicht geeignet ist.

Dementsprechend umfasst eine bevorzugte Vorrichtung als Wundkontaktschicht ein Hydrogel, das seinerseits bei der Behandlung von nässenden Wunden oder auch chronischen Wunden, zum Beispiel Dekubitus-Geschwüren, besonders vorteilhaft ist.

Eine erfindungsgemäße Vorrichtung kann auch eine Wundauflage umfassen, die eine Wundkontaktschicht aufweist, die mindestens ein Hydrocolloid umfasst. Gemäß der vorliegenden Erfindung soll unter einem Hydrocolloid ein Material verstanden sein, das ein hydrophiles synthetisches oder natürliches Polymermaterial ist, das löslich oder absorbierend und/ oder quellend in Wasser ist. Vorzugsweise umfasst eine Wundkontaktschicht ein Hydrocolloid aus einem synthetischen oder natürlichen Polymermaterial, das ausgewählt wird aus der Gruppe Alginsäure und deren Salze sowie deren Derivate, Chitin oder dessen Derivate, Chitosan oder dessen Derivate, Pektin, Cellulose oder dessen Derivate wie Celluloseether oder Celluloseaster, vernetzte oder nicht vernetzte Carboxyalkylcellulose oder Hydroxyalkylcellulose, Polyvinylalkohol, Polyvinylpyrrolidon, Agar, Guargum oder Gelatine.

Das Hydrocolloid kann sowohl in Form von Fasern als auch in Form von Partikeln und/ oder Fasern innerhalb einer Matrix vorliegen. Insbesondere kann das Hydrocolloid in Form von Partikeln in einer klebenden Polymermatrix vorliegen. Die klebende Polymermatrix umfasst dabei mindestens ein Co-Blockpolymer ausgewählt aus der Gruppe der AB-Diblock-Copolymere und/ oder ABA-Triblock-Copolymere, das aus den Monomeren Styrol, Butadien und Isopren aufgebaut ist. Der Anteil an Hydrocolloidpartikel in der Wundkontaktschicht kann vorzugsweise 10 bis 70 Gew.-% bezogen auf das Gesamtgewicht der Wundkontaktschicht aufweisen. Eine solche Zusammensetzung ist zum Beispiel mit der EP 1 007 597 B1 bekannt.

Dementsprechend umfasst eine bevorzugte Vorrichtung als Wundkontaktschicht eine selbstklebende Polymermatrix, in die Hydrocolloidpartikel dispergiert sind. Eine solche Vorrichtung kann insbesondere bei der Behandlung von stark nässenden Wunden oder auch chronischen Wunden, zum Beispiel bei Dekubitus-Geschwüren, eingesetzt werden.

Es kann weiterhin durch die Wundkontaktschicht eine die Wundheilung fördernde Substanz freigesetzt werden. Hierzu zählen insbesondere Substanzen die fungizid, bakterizid oder antimikrobiell wirken. In einer besonderen Ausführungsform umfasst die Wundkontaktschicht ein Hydrogel oder ein Hydrocolloid, das seinerseits mindestens eine fungizid, bakterizid oder antimikrobiell wirkende Substanz umfasst. Ganz besonders sind hierbei Chitosan, Silber, Silberkomplexe, Silbersalze, Zink, Zinksalze oder Zinkkomplexe geeignet.

Es kann weiterhin vorgesehen sein, das eine erfindungsgemäße Vorrichtung eine Wundauflage umfasst, die alternativ oder zusätzlich weitere Schichten aufweist, die unterschiedliche Funktionen besitzen. Gemäß einer Weiterntwicklung der vorliegenden Erfindung weist die Wundauflage mindestens eine weitere Schicht auf. Diese Schicht kann bevorzugt eine Releaseschicht zum Schutz vor Verschmutzungen sein, die auf der im anwendungsgerechten Zustand der Vorrichtung auf der Wunde zu liegenden Seite der Wundkontaktschicht aufgebracht ist. Es kann auch vorgesehen sein, dass die Wundauflage mindestens eine weitere Schicht zwischen der Wundkontaktschicht und der Trägerschicht aufweist. Die weitere Schicht kann sowohl eine absorbierende Schicht als auch eine eine Flüssigkeit aufnehmende und über die Fläche der Wundauflage verteilende Schicht sein. Dem gemäß umfasst eine bevorzugte Vorrichtung eine Wundauflage, die ihrereseits eine Trägerschicht, eine Wundkontaktschicht und eine zwischen der Trägerschicht und der Wundkontaktschicht angeordnete Verteilerschicht umfasst.

In einer besonderen Ausgestaltung der Erfindung ist vorgesehen, dass eine erfindungsgemäße Vorrichtung in einer Verpackung angeordnet ist. Insbesondere ist dabei vorgesehen, dass die Verpackung eine sterile Verpackung ist.

Schließlich betrifft die vorliegende Erfindung ein Verfahren, mit dem die hier beschriebenen Ausführungsformen hergestellt werden können. Insbesondere ein Verfahren zur Herstellung einer Vorrichtung, die eine Wundauflage zur Behandlung einer Wunde oder der die Wunde umgebenden Haut und mindestens eine Wunddokumentationshilfe zur Dokumentation von charakteristischen Daten der Wunde oder der die Wunde umgebende Haut umfasst, wobei die Wundauflage mindestens eine die Wundheilung fördernde Wundkontaktschicht und eine Trägerschicht aufweist, die Wundauflage mit der Wunddokumentationshilfe lösbar verbunden ist und die Wunddokumentationshilfe mindestens eine Folie umfasst. Das Verfahren umfasst dabei die Verfahrensschritte:
a) Herstellung eines Schichtverbundes bestehend aus der Trägerschicht und der Wunddokumentationshilfe umfassend eine Folie und
b) Aufbringen der Wundkontaktschicht auf die der Wunddokumentationshilfe abgewandte Seite der Trägerschicht.

Vorzugsweise erfolgt die Herstellung des Schichtverbundes unter a) bei gleichzeitigem Anbringen einer Griffleiste an die Folie der Dokumentationshilfe. Diese Griffleiste umfasst ihrerseits ein doppelseitig klebend ausgerüstetes Klebeband, das mit einem Abdeckstreifen auf einer Seite abgedeckt ist. Die nicht abgedeckte Seite des Klebebandes wird auf die der Trägerschicht zugewandten Seite der Folie der Dokumentationshilfe und/ oder auf die der Trägerschicht abgewandten Seite der Folie der Dokumentationshilfe aufgebracht. In einem weiteren bevorzugten Verfahren wird eine "in-line"-Bedruckung der Folie vorgenommen. Diese Bedruckung erfolgt vor dem Herstellen des Schichtverbundes a) auf der der Trägerschicht abgewandten Seite der Folie der Dokumentationshilfe.

In einem alternativen Verfahren zur Herstellung einer erfindungsgemäßen Vorrichtung umfassend eine Wundauflage zur Behandlung einer Wunde oder der die Wunde umgebenden Haut und mindestens eine Wunddokumentationshilfe zur Dokumentation von charakteristischen Daten der Wunde oder der die Wunde umgebende Haut, wobei die Wundauflage mindestens eine die Wundheilung fördernde Wundkontaktschicht und eine Trägerschicht aufweist, die Wundauflage mit der Wunddokumentationshilfe lösbar verbunden ist und die Wunddokumentationshilfe mindestens eine Folie umfasst, werden die Verfahrensschritte
a) Bereitstellen einer Wundauflage umfassend mindestens eine Trägerschicht und eine Wundkontaktschicht und
b) Aufbringen der Folie der Wunddokumentationshilfe auf die der Wundkontaktschicht abgewandte Seite der Trägerschicht
verwirklicht.

Neben einer Vorrichtung ist weiterhin ein Verfahren zur Dokumentation der Wundgröße oder anderer charakteristischer Kenngrößen einer Wunde Gegenstand der vorliegenden Erfindung. Das erfindungsgemäße Verfahren umfasst die Verfahrensschritte
a) Applizieren einer Vorrichtung gemäß mindestens einer der beschriebenen Ausführungsformen auf einer Wunde und die der Wunde umgebenden Haut und
b) Kennzeichnen der Wundgröße oder anderer charakteristischer Kenngrößen mittels der Dokumentationshilfe.

Verfahren zur Dokumentation der Wundgröße oder anderer charakteristischer Kenngrößen einer Wunde mit den Verfahrensschritten
a) Verwendung einer Vorrichtung nach mindestens einem der vorgenannten Ansprüche zur Herstellung eines Mittels zur Behandlung der Wunde und
b) Kennzeichnen der Wundgröße oder anderer charakteristischer Kenngrößen mittels der Dokumentationshilfe.

Gemäß einer Weiterbildung des Verfahrens ist in einem weiteren Verfahrensschritt c) eine Archivierung der Dokumentationshilfe vorgesehen, wobei die Wundauflage auf der Wunde verbleibt.

Darüber hinaus ist die Verwendung einer erfindungsgemäßen Vorrichtung zur Herstellung eines Mittels zur Dokumentation von Wunden, chronischen Wunden oder Hautschädigungen besonders geeignet.

Hervorzuheben ist an dieser Stelle, dass die hier aufgeführten Merkmale der alternativen Ausgestaltungen der Erfindungen nicht auf die einzelnen Alternativen zu beschränken sind. Es ist vielmehr der Fall, dass die Kombinationen der Ausgestaltungen bzw. die Kombinationen der Einzelmerkmale der alternativen Formen ebenso erfindungsgemäße Ausgestaltungen bilden.

Nachstehend wird die Erfindung unter Bezugnahme auf die Zeichnung erläutert. In den Zeichnungen zeigt:
- Figur 1:: eine Dokumentationshilfe in Aufsicht
- Figur 2:: eine erfindungsgemäße Vorrichtung in Aufsicht
- Figur 3:: die in Figur 2 wiedergegebene Ausführungsform einer Vorrichtung im Querschnitt
- Figur 4:: eine weitere Ausführungsform einer Vorrichtung im Querschnitt
- Figur 5:: eine weitere Ausführungsform einer Vorrichtung im Querschnitt
- Figur 6:: eine weitere Ausführungsform einer Vorrichtung im Querschnitt

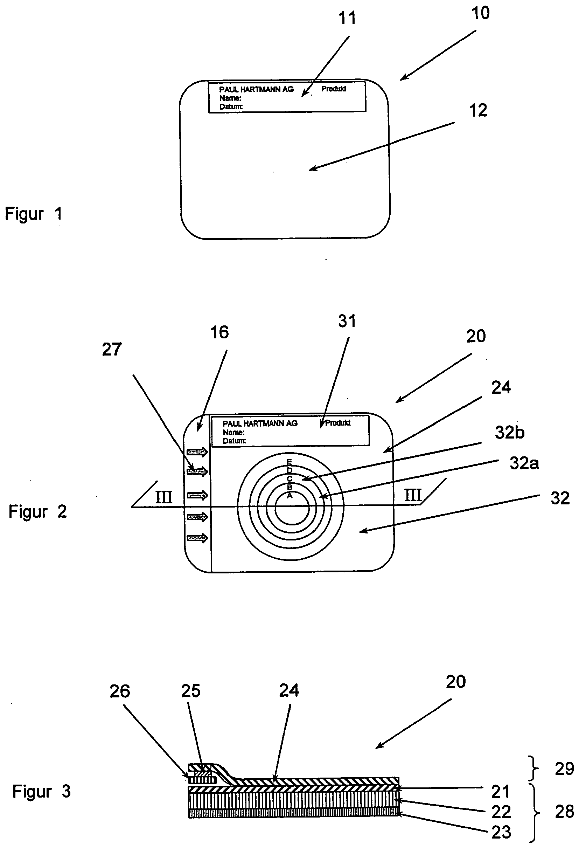

Mit Figur 1 ist eine Dokumentationshilfe in Form einer transparenten Folie (10) wiedergegeben. Die Folie besteht aus Polyethylen und besitzt eine glatte Oberfläche, die mit einem Stift z.B. Permanent-Marker beschreibbar ist. Die Folie weist ein erstes Dokumentationsfeld (11) und ein zweites Dokumentationsfeld (12) auf. In das erste Dokumentationsfeld (11) kann z.B. die Art der Maßnahme in Form des Produktnamens, das Datum der Anwendung und der Name des Patienten aufgenommen werden. In das zweite Dokumentationsfeld (12) kann z.B. die Art der Wunde oder der Wundumriss aufgezeichnet werden.

Mit Figur 2 und 3 ist eine erfindungsgemäße Vorrichtung (20) wiedergegeben. Dabei weist die Vorrichtung eine Wundauflage (28) bestehend aus einer Trägerschicht (21) aus einem dünnen Polyurethanfilm, eine Wundkontaktschicht (22) aus einem Polyurethangel und eine die Wundkontaktschicht abdeckende Releaseschicht (23) aus einem ersten silikonisierten Releasepapier auf. Auf der Trägerschicht (21) ist eine Wunddokumentationshilfe (29) aufgebracht, die eine beschreibbare Polyethylenfolie (24), einen adhäsiven Streifen (25) und ein den adhäsiven Streifen abdeckendes zweites silikonisiertes Releasepapier (26) aufweist. Die Folie (24) ist dabei mit der Trägerschicht (21) direkt verbunden. Die Bindungskräfte zwischen der Folie (24) und der Trägerschicht (21) sind dabei so eingestellt, dass die Dokumentationshilfe (29) mit der Hand leicht von der Wundauflage (28) entfernbar ist. Lediglich im Bereich des zweiten Releasepapiers (26) ist die Dokumentationshilfe (29) ohne jede Bindung mit der Wundauflage (28) versehen. Somit kann auch dieser abgedeckte Bereich gleichzeitig als Griffleiste zur leichteren Ergreifung der Dokumentationshilfe bei der Entfernung von der Wundauflage eingesetzt werden. Ein Druckbild (27) auf dem Releasepapier (26), das auf der der Folie (24) zugewandten Seite angebracht ist, soll bei Gebrauch die Abzugsrichtung der Dokumentationshilfe (29) anzeigen. Die Dokumentationshilfe (29) weist zwei Dokumentationsfelder (31, 32) auf. Das erste Dokumentationsfeld (31) ermöglicht die Feststellung der durchgeführten therapeutischen Maßnahme, wogegen das zweite Dokumentationsfeld (32) die Dokumentation des Ist-Zustandes der Wunde erlaubt. Zur Erleichterung der Feststellung der Wundgröße sind in dem zweiten Dokumentationsfeld konzentrische Kreise (32a) mit zugeordneten Bezeichnungen (32b) angebracht. Zur Dokumentation kann der Anwender den Wundrand in dem zweiten Dokumentationsfeld aufzeichnen und gleichzeitig anhand der Bezeichnungen eine Kategorisierung der Wunde vornehmen. Somit erlaubt die Verwendung dieser Vorrichtung auch ein standardisiertes Verfahren zur Kennzeichnung der Wundgröße. Nachdem die Dokumentationshilfe von der Wundauflage entfernt worden ist, kann der Anwender das zweite Releasepapier (26) von dem adhäsiven Streifen (25) entfernen und die Dokumentationshilfe mittels des adhäsiven Streifens in eine Patientenakte einkleben.

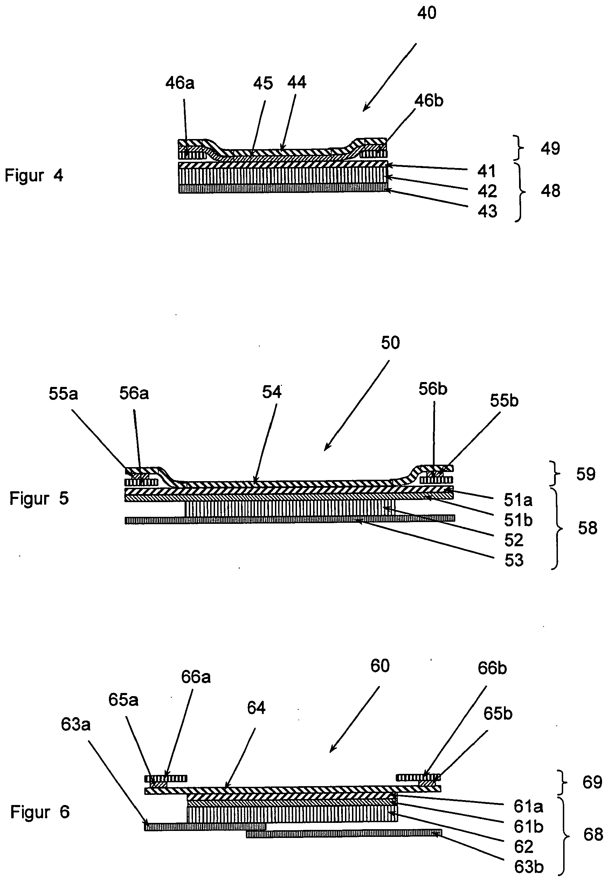

Mit Figur 4 ist eine zweite Ausführung einer erfindungsgemäßen Vorrichtung wiedergegeben. Die Vorrichtung (40) weist dabei eine Wundauflage (48) bestehend aus einer Trägerschicht (41) aus Polyetherurethan, eine Wundkontaktschicht (42), die eine selbstklebende Matrix umfasst, in die Hydrocolloidpartikel dispergiert sind, und eine die selbstklebende Matrix abdeckende Releaseschicht (43) aus einem silikonisierten Releasepapier auf. Die Dokumentationshilfe (49) besteht aus einer Polypropylen-Folie (44), einem Dispersionshaftkleber (45) und zwei fluorierten und damit ablösbaren Abdeckstreifen (46a, 46b) und ist vollflächig bis auf zwei Randbereiche, die mittels der Abdeckstreifen (46a, 46b) abgedeckt sind, mit der Trägerschicht (41) verbunden. Die beiden abgedeckten Bereiche können wiederum als Griffleiste verwendet werden. Nach dem Applizieren der Vorrichtung auf der zu behandelnden Wunde kann kontaminationsfrei eine Aufzeichnung der Wund-Kenngrößen auf der Dokumentationshilfe mittels eines Permanent-Markers vorgenommen werden. Die Aufzeichnung und Dokumentation erfolgt hierbei sehr reizarm, da einerseits die Wundkontaktschicht die Folie von der Wunde beabstandet und andererseits die Dokumentationshilfe ohne großen Kraftaufwand und damit ohne Verschiebung der Wundauflage auf der Wunde erfolgen kann.

Mit Figur 5 ist eine dritte Ausführung einer erfindungsgemäßen Vorrichtung wiedergegeben. Die Vorrichtung (50) liegt in quadratischer Form vor (hier nicht dargestellt) und ist als Inseldressing gefertigt, das heißt, dass die Trägerschicht (51 a) allseitig über die Wundkontaktschicht (52) hinausragt. Die Wundauflage (58) besteht dabei aus einem transparenten, wasserdampfdurchlässigen Polyurethanfilm (51a), der vollflächig mit einem Haftkleber (51 b) beschichtet ist, einer Wundkontaktschicht (52) aus einem Alginatfaservlies, das mittel des Haftklebers an der Trägerschicht fixiert und einer ersten silikonisierten Releasepapierschicht (53), die sowohl die Wundkontaktschicht (52) als auch die klebenden Ränder der Trägerschicht (51 a) abgedeckt. Auf der Trägerschicht ist direkt eine Dokumentationshilfe in Form einer Folie aufgebracht. Die Dokumentationshilfe (59) besteht dabei aus einer beschreibbaren, transparenten Polyethylenfolie (54), zwei adhäsiven Streifen (55a, 55b), die parallel, an zwei sich gegenüberliegenden Rändern der Folie angebracht sind, und zwei diese adhäsiven Streifen abdeckende Releasepapierstreifen (56a, 56b). Die Folie ist bis auf die Bereiche, die durch die Releasepapierstreifen (56a, 56b) abgedeckt sind, vollständig mit der Trägerschicht verbunden. Insgesamt weist die Dokumentationshilfe denselben Umfang auf, wie die Wundauflage.

Mit Figur 6 ist eine weitere erfinderische Ausführung einer Vorrichtung gezeigt. Diese Vorrichtung (60) besteht aus einer Wundauflage (68), die eine Trägerschicht (61 a) aus einem transparenten Polyesterpolyurethan, die mit einem wasserdampfdurchlässigen Polyurethanhaftkleber (61 b) vollflächig beschichtet ist, eine Wundkontaktschicht (62) aus einem gefriergetrockneten Hydrogelschaum und eine zweigeteilte Releaseschicht (63a, 63b) aus Polyethylenfolie aufweist. Die Trägerschicht (61) weist dieselbe Umfangsbegrenzung wie die Wundkontaktschicht auf. Auf der Trägerschicht ist eine Wunddokumentationshilfe (69) aufgebracht. Die Dokumentationshilfe (69) besteht aus einer Polyesterfolie (64) zwei adhäsive Streifen (65a, 65b) und zwei Releasepapierstreifen (66a, 66b). Die adhäsiven Streifen (65a, 65b) liegen auf derselben Seite auf der auch die Kenngrößen der Wunde aufgenommen werden können. Damit kann selbst bei der Verwendung eines nicht-permanten Markers eine dokumentationssichere Aufzeichnung der Kenngrößen vorgenommen werden, da eben die Seite mit den Aufzeichnungen auf die Dokumentakte fixiert werden kann. Um die Dokumentationshilfe von der Wundauflage zu entfernen, bedient sich der Anwender der beiden Releasepapierstreifen (66a, 66b), die eine freie Anfassfläche als Griffleiste aufweisen. Dabei sind die Releasekräfte zwischen der Trägerschicht und der Folie derart eingestellt, dass die Kraft, die notwendig ist, um eben diese voneinander zu trennen, geringer ist als die Kraft, die notwendig ist, um einen Releasepapierstreifen (66a, 66b) von einem adhäsiven Streifen (65a, 65b) zu trennen. Auf der Folie ist zudem zur leichteren Charakterisierung der Wunde ein Gitter aufgebracht, das Gitterfelder von 1 cm² aufweist (hier nicht dargestellt). Die Trägerschicht weist ebenfalls diese Gitterfelder auf und die Linien der Gitterfelder sind gleich denen der Dokumentationshilfe angeordnet. So kann der Anwender, der eine solche Vorrichtung nutzt, zu jeder Zeit eine gleiche Kenngrößenbestimmung der Wunde vornehmen ohne die Wundauflage zu wechseln.

### Beispiel 1

Ein Ausführungsbeispiel stellt eine Hydrokolloidwundauflage dar, die mit einer Wunddokumentationsfolie versehen ist. Dabei weist die Vorrichtung einen Aufbau analog Figur 4 auf. Die Wundauflage (48) wird hergestellt, indem auf einen Lagenverbund aus einem Polyurethanfilm und einer Hydrokolloidschicht (MED 5586H der Firma AveryDennison) auf der selbstklebenden Seite der Hydrokolloidschicht ein Silikonpapier (Separacon 9120-60 der Firma Maria Soell, Flächengewicht 120 g/m2) als Schutzpapier aufgebracht wird. Auf die Polyurethanfolie (41) wird ein selbstklebend ausgerüsteter Polyethylenfilm (44 und 45) der Firma 3M (3M 5504A-UV) als Wunddokumentationshilfe (49) auflaminiert. Der beschreibbare Polyethylenfilm (44) besitzt eine Dicke von 56 µm und ist mit einem Acrylatkleber (45) beschichtet, so dass eine Klebkraft gemäß AFERA 4001 von 0,4 N/cm resultiert. Auf die selbstklebend ausgerüstete Seite des Polyethylenfilms (die zur PU-Folie des MED 5586H-Materials zeigt) wird auf mindestens einer Längsseite ein silikonisierter Papierstreifen (46a, 46b) (z.B. Separacon 9120-60 der Firma Maria Soell, Flächengewicht 120 g/m2) bzw. ein mit einem Silikonpapier abgedecktes doppelseitiges Klebeband auflaminiert. Diese dienen als Griffleiste zur leichteren Entfernung der Dokumentationsfolie von der Wundauflage und können vor dem Einkleben in die Patientendokumentation von der Folie entfernt werden. Besonders im Falle des doppelseitigen Klebebandes führt dies zu einer verbesserten Klebkraft der Dokumentationsfolie in der Patientenakte.

### Beispiel 2

Ein weiteres Ausführungsbeispiel umfasst eine Hydrogelwundauflage, die mit einer transparenten Dokumentationsfolie ausgerüstet ist. Dabei weist die Vorrichtung analog Figur 3 eine Wundauflage (28) bestehend aus einer Trägerschicht (21) aus einem dünnen Polyurethanfilm (Firma Gerlinger Type Estane 5707), eine Wundkontaktschicht (22) aus einem Polyurethangel und eine die Wundkontaktschicht abdeckende Releaseschicht (23) aus einem ersten silikonisierten Releasepapier (LDPE-Folie SKF 1002) auf. Das Polyurethangel besteht (analog EP 426 422 B1) aus 16,5 Gew.-% Polypropylenglycol (Fa. DOW; Schwalbach, (Deutschland)), 12,5 Gew.-% Präpolymer mit Isophorondiisocyanat-Enden (Aquapol 302-0031; Fa. Carpenter Co., Richmon (USA),), 8,7 Gew.-% Diamin auf Polyethylenoxidbasis (Jeffamine ED 2003; Fa. Huntsman, Rotterdam (Niederlande)), 1 Gew.-% Natriumchlorid und 61,3 Gew.-% demineralisiertes Wasser. Auf der Trägerschicht (21) ist eine Wunddokumentationshilfe (29) aufgebracht, die eine beschreibbare Polyethylenfolie (24) (Bulken-SH-Folie Type 5018 SHE), einen adhäsiven Streifen (25) und ein den adhäsiven Streifen abdeckendes zweites silikonisiertes Releasepapier (26) aufweist. Dieses doppelseitige Klebeband (25, 26) ist von der Firma Gerlinger unter der Bezeichnung (Dokutape) erhältlich. Die Folie (24) ist dabei mit der Trägerschicht (21) direkt verbunden. Lediglich im Bereich des zweiten Releasepapiers (26) ist die Dokumentationshilfe (29) ohne jede Bindung mit der Wundauflage (28) versehen. Somit kann auch dieser abgedeckte Bereich gleichzeitig als Griffleiste zur leichteren Ergreifung der Dokumentationshilfe bei der Entfernung von der Wundauflage eingesetzt werden. Ein Druckbild (27) auf dem Releasepapier (26), der auf der der Folie (24) zugewandten Seite angebracht ist, soll bei Gebrauch die Abzugsrichtung der Dokumentationshilfe (29) anzeigen. Die Dokumentationshilfe (29) weist zwei Dokumentationsfelder (31,32) auf. Das erste Dokumentationsfeld (31) ermöglicht die Feststellung der durchgeführten therapeutischen Maßnahme, wogegen das zweite Dokumentationsfeld (32) die Dokumentation des Ist-Zustandes der Wunde erlaubt. Nachdem die Dokumentationshilfe von der Wundauflage entfernt worden ist, kann der Anwender das zweite Releasepapier (26) von dem adhäsiven Streifen (25) entfernen und die Dokumentationshilfe mittels des adhäsiven Streifens in eine Patientenakte einkleben.

## Patentansprüche

1. Vorrichtung (20, 40, 50, 60) umfassend eine Wundauflage (28, 48, 58, 68) zur Behandlung einer Wunde oder der die Wunde umgebenden Haut und mindestens eine Wunddokumentationshilfe (29, 49, 59, 69) zur Dokumentation von charakteristischen Daten der Wunde oder der die Wunde umgebenden Haut, wobei die Wundauflage mindestens eine die Wundheilung fördernde Wundkontaktschicht (22, 42, 52, 62) und eine Trägerschicht (21, 41, 51, 61 a) aufweist, die Wundauflage (28, 48, 58, 68) mit der Wunddokumentationshilfe (29, 49, 59, 69) lösbar verbunden ist und die Wundauflage (28, 48, 58, 68) transparent oder transluzent ist, **dadurch gekennzeichnet, dass** die Wunddokumentationshilfe (29, 49, 59, 69) mindestens einen klebend ausgerüsteten Bereich aufweist, der mittels eines Abdeckstreifens (26, 46a, 46b, 56a, 56b, 66a, 66b) abgedeckt ist, und mindestens eine Griffleiste aufweist, wobei der mittels eines Abdeckstreifens (26, 46a, 46b, 56a, 56b, 66a, 66b) abgedeckte, klebend ausgerüstete Bereich als Griffleiste verwendbar ist.

2. Vorrichtung nach Anspruch 1 **dadurch gekennzeichnet, dass** die Wunddokumentationshilfe (29, 49, 59, 69) mindestens eine beschreibbare Folie (24, 44, 54, 64) umfasst.

3. Vorrichtung nach Anspruch 1 oder 2 **dadurch gekennzeichnet, dass** die Wunddokumentationshilfe (29, 49, 59, 69) mindestens zwei Dokumentationsfelder (11, 12, 31, 32) zur Speicherung von Informationen umfasst.

4. Vorrichtung nach mindestens einem der vorgenannten Ansprüche **dadurch gekennzeichnet, dass** die Dokumentationshilfe (29, 49, 59, 69) direkt mit der Trägerschicht lösbar verbunden ist.

5. Vorrichtung nach mindestens einem der vorgenannten Ansprüche **dadurch gekennzeichnet, dass** die Wundkontaktschicht (22, 42, 52, 62) eine auf der im anwendungsgerechten (23, 43, 53, 63) Zustand auf der Wunde zu liegen kommenden Seite eine Abdeckschicht (23, 43, 53, 63a, 63b) aufweist.

6. Vorrichtung nach mindestens einem der vorgenannten Ansprüche **dadurch gekennzeichnet, dass** die Wundauflage (28, 48, 58, 69) als Wundkontaktschicht (22, 42, 52, 62) mindestens ein Hydrogel, ein Hydrocolloid oder einen Schaum umfasst.

7. Vorrichtung nach mindestens einem der vorgenannten Ansprüche **dadurch gekennzeichnet, dass** die Wundauflage (28, 48, 58, 68) als Wundkontaktschicht (22, 42, 52, 62) ein Hydrogel umfasst.

8. Vorrichtung nach mindestens einem der vorgenannten Ansprüche **dadurch gekennzeichnet, dass** die Wundauflage (28, 48, 58, 68) als Wundkontaktschicht (22, 42, 52, 62) eine selbstklebende Polymermatrix umfasst, in die Hydrocolloidpartikel dispergiert sind.

9. Vorrichtung nach mindestens einem der vorgenannten Ansprüche **dadurch gekennzeichnet, dass** die Wundauflage (28, 48, 58, 68) als Trägerschicht (21, 41, 51, 61) einen Film aus Polyurethan, Polyesterurethan oder Polyetherurethan aufweist.

10. Vorrichtung nach mindestens einem der vorgenannten Ansprüche **dadurch gekennzeichnet, dass** die Wundauflage (28, 48, 58, 68) dieselbe Umfangsbegrenzung aufweist wie die Wunddokumentationshilfe (29, 49, 59, 69).

11. Verfahren zur Dokumentation der Wundgröße oder anderer charakteristischer Kenngrößen einer Wunde mit den Verfahrensschritten
a) Applizieren einer Vorrichtung (20, 40, 50, 60) nach mindestens einem der vorgenannten Ansprüche auf die Wunde und
b) Kennzeichnen der Wundgröße oder anderer charakteristischer Kenngrößen mittels der Dokumentationshilfe.

12. Verfahren nach Anspruch 11 **dadurch gekennzeichnet, dass** als weiterer Verfahrensschritt c) eine Archivierung der Dokumentationshilfe erfolgt, wobei die Wundauflage auf der Wunde verbleibt.

13. Verwendung einer Vorrichtung (20, 40, 50, 60) nach mindestens einem der vorgenannten Ansprüche zur Dokumentation von Wunden, chronischen Wunden oder Hautschädigungen.

## Claims

1. Device (20, 40, 50, 60) comprising a wound dressing (28, 48, 58, 68), for treating a wound or the skin surrounding the wound, and at least one wound documentation aid (29, 49, 59, 69), for documenting characteristic data of the wound or of the skin surrounding the wound, the wound dressing having at least one wound contact layer (22, 42, 52, 62), which promotes the healing of the wound, and a support layer (21, 41, 51, 61a), the wound dressing (28, 48, 58, 68) being connected detachably to the wound documentation aid (29, 49, 59, 69), and the wound dressing (28, 48, 58, 68) being transparent or translucent, **characterized in that** the wound documentation aid (29, 49, 59, 69) has at least one adhesive area, which is covered by means of a cover strip (26, 46a, 46b, 56a, 56b, 66a, 66b), and has at least one grip tab, the adhesive area covered by means of a cover strip (26, 46a, 46b, 56a, 56b, 66a, 66b) being able to be used as grip tab.

2. Device according to Claim 1, **characterized in that** the wound documentation aid (29, 49, 59, 69) comprises at least one inscribable film (24, 44, 54, 64) .

3. Device according to Claim 1 or 2, **characterized in that** the wound documentation aid (29, 49, 59, 69) comprises at least two documentation fields (11, 12, 31, 32) for storing information.

4. Device according to at least one of the preceding claims, **characterized in that** the documentation aid (29, 49, 59, 69) is detachably connected to the support layer directly.

5. Device according to at least one of the preceding claims, **characterized in that** the wound contact layer (22, 42, 52, 62) has a covering layer (23, 43, 53, 63a, 63b) on the side that comes to lie on the wound in the correct state of use (23, 43, 53, 63).

6. Device according to at least one of the preceding claims, **characterized in that** the wound dressing (28, 48, 58, 69) comprises, as wound contact layer (22, 42, 52, 62), at least a hydrogel, a hydrocolloid or a foam.

7. Device according to at least one of the preceding claims, **characterized in that** the wound dressing (28, 48, 58, 68) comprises a hydrogel as wound contact layer (22, 42, 52, 62).

8. Device according to at least one of the preceding claims, **characterized in that** the wound dressing (28, 48, 58, 68) comprises, as wound contact layer (22, 42, 52, 62), a self-adhesive polymer matrix in which hydrocolloid particles are dispersed.

9. Device according to at least one of the preceding claims, **characterized in that** the wound dressing (28, 48, 58, 68) has, as support layer (21, 41, 51, 61), a film of polyurethane, polyester urethane or polyether urethane.

10. Device according to at least one of the preceding claims, **characterized in that** the wound dressing (28, 48, 58, 68) has the same peripheral boundary as the wound documentation aid (29, 49, 59, 69).

11. Method for documenting the size of a wound or other characteristic parameters of a wound, with the method steps of
a) applying a device (20, 40, 50, 60) according to at least one of the preceding claims to the wound, and
b) indicating the size of the wound or other characteristic parameters by means of the documentation aid.

12. Method according to Claim 11, **characterized in that**, as a further method step c), the documentation aid is archived, with the wound dressing remaining on the wound.

13. Use of a device (20, 40, 50, 60) according to at least one of the preceding claims for documenting wounds, chronic wounds or skin damage.

## Revendications

1. Dispositif (20, 40, 50, 60) comprenant un pansement (28, 48, 58, 68) pour le traitement d'une plaie ou de la peau entourant une plaie et au moins un auxiliaire de documentation de plaie (29, 49, 59, 69) pour la documentation de données caractéristiques de la plaie ou de la peau entourant la plaie, dans lequel le pansement présente au moins une couche de contact de plaie (22, 42, 52, 62) favorisant la cicatrisation de la plaie et une couche de support (21, 41, 51, 61a), le pansement (28, 48, 58, 68) est attaché de façon séparable à l'auxiliaire de documentation de plaie (29, 49, 59, 69) et le pansement (28, 48, 58, 68) est transparent ou translucide, **caractérisé en ce que** l'auxiliaire de documentation de plaie (29, 49, 59, 69) présente au moins une zone à caractère adhésif, qui est recouverte d'une bande de recouvrement (26, 46a, 46b, 56a, 56b, 66a, 66b), et présente au moins un onglet, dans lequel la zone à caractère adhésif recouverte d'une bande de recouvrement (26, 46a, 46b, 56a, 56b, 66a, 66b) peut être utilisée comme onglet.

2. Dispositif selon la revendication 1, **caractérisé en ce que** l'auxiliaire de documentation de plaie (29, 49, 59, 69) comprend au moins une feuille écrivable (24, 44, 54, 64).

3. Dispositif selon la revendication 1 ou 2, **caractérisé en ce que** l'auxiliaire de documentation de plaie (29, 49, 59, 69) comprend au moins deux champs de documentation (11, 12, 31, 32) pour le stockage d'informations.

4. Dispositif selon au moins une des revendications précédentes, **caractérisé en ce que** l'auxiliaire de documentation de plaie (29, 49, 59, 69) est attaché de façon séparable directement à la couche de support.

5. Dispositif selon au moins une des revendications précédentes, **caractérisé en ce que** la couche de contact de plaie (22, 42, 52, 62) présente une couche de recouvrement (23, 43, 53, 63a, 63b) sur le côté à appliquer sur la plaie dans l'état prêt à l'emploi (23, 43, 53, 63)

6. Dispositif selon au moins une des revendications précédentes, **caractérisé en ce que** le pansement (28, 48, 58, 69) comprend comme couche de contact de plaie (22, 42, 52, 62) au moins un hydrogel, un hydrocolloïde ou une mousse.

7. Dispositif selon au moins une des revendications précédentes, **caractérisé en ce que** le pansement (28, 48, 58, 68) comprend un hydrogel comme couche de contact de plaie (22, 42, 52, 62).

8. Dispositif selon au moins une des revendications précédentes, **caractérisé en ce que** le pansement (28, 48, 58, 68) comprend comme couche de contact de plaie (22, 42, 52, 62) une matrice polymère autoadhésive, dans laquelle des particules d'hydrocolloïde sont dispersées.

9. Dispositif selon au moins une des revendications précédentes, **caractérisé en ce que** le pansement (28, 48, 58, 68) présente comme couche de support (21, 41, 51, 61) un film en polyuréthane, en polyester uréthane ou en polyéther uréthane.

10. Dispositif selon au moins une des revendications précédentes, **caractérisé en ce que** le pansement (28, 48, 58, 68) présente la même limitation périphérique que l'auxiliaire de documentation de plaie (29, 49, 59, 69).

11. Procédé pour la documentation de la grandeur de plaie ou d'autres paramètres caractéristiques d'une plaie comprenant les étapes suivantes:
a) appliquer un dispositif (20, 40, 50, 60) selon au moins une des revendications précédentes sur la plaie, et
b) caractériser la grandeur de la plaie ou d'autres paramètres caractéristiques au moyen de l'auxiliaire de documentation.

12. Procédé selon la revendication 11, **caractérisé en ce que** l'on effectue comme autre étape du procédé c) un archivage de l'auxiliaire de documentation, dans lequel le pansement reste sur la plaie.

13. Utilisation d'un dispositif (20, 40, 50, 60) selon au moins une des revendications précédentes pour la documentation de plaies, de plaies chroniques ou de lésions cutanées.
